(19) Europäisches Patentamt
European Patent Office
Office européen des brevets

(11) Veröffentlichungsnummer: **0 008 763**
**B1**

(12) # EUROPÄISCHE PATENTSCHRIFT

(45) Veröffentlichungstag der Patentschrift: **01.04.81**

(21) Anmeldenummer: **79103157.8**

(22) Anmeldetag: **27.08.79**

(51) Int. Cl.³: **C 07 C 87/34,** C 10 L 1/10, C 10 M 1/32, C 10 M 3/26, C 10 M 5/20

(54) **Aminomethyl-cyclododecane, ein Verfahren zu ihrer Herstellung und ihre Verwendung als Korrosionsschutzmittel.**

(30) Priorität: **06.09.78 DE 2838755**

(43) Veröffentlichungstag der Anmeldung: **19.03.80 Patentblatt 80/6**

(45) Bekanntmachung des Hinweises auf die Patenterteilung: **01.04.81 Patentblatt 81/13**

(84) Benannte Vertragsstaaten: **BE CH DE FR GB IT NL**

(56) Entgegenhaltungen:
**DE-A-1 964 841**
**DE-A1-2 515 486**
**DE-A1-2 614 244**
**DE-B-1 668 255**
**DE-B-1 807 827**
**GB-A-1 170 226**
**US-A-3 342 878**
**US-A-3 499 932**
**US-A-3 499 933**
**US-A-3 804 914**
**US-B-316 917**

(73) Patentinhaber: **BAYER AG, Zentralbereich Patente, Marken und Lizenzen, D-5090 Leverkusen 1, Bayerwerk (DE)**

(72) Erfinder: **Braden, Rudolf, Dr., Nothauser Feld 1, D-5068 Odenthal (DE)**
Erfinder: **Wagner, Kuno, Dr., Am Kiesberg 8, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).



ACTORUM AG.

# Aminomethyl-cyclododecane, ein Verfahren zu ihrer Herstellung und ihre Verwendung als Korrosionsschutzmittel

Gegenstand der vorliegenden Erfindung sind Aminomethylcyclododecane ausgewählt aus der Gruppe bestehend aus Aminomethyl-cyclododecan, Bis-(aminomethyl)-cyclododecanen, Tris-(aminomethyl)-cyclododecanen und deren Gemischen.

Gegenstand der vorliegenden Erfindung ist auch ein Verfahren zur Herstellung dieser neuen Verbindungen, welches dadurch gekennzeichnet ist, dass man Cyclododecatrien-1,5,9 in Gegenwart eines Rhodium-haltigen Katalysators mit Kohlenmonoxid und Wasserstoff bei Temperaturen von 80 bis 180 °C und Drucken von 30 bis 900 bar umsetzt, den Katalysator von dem Hydroformulierungsprodukt abtrennt und die Hydroformulierungsprodukte, gegebenenfalls nach ihrer destillativen Auftrennung in die einzelnen Komponenten, in Gegenwart von Ammoniak und einem Hydrierkatalysator bei 50 bis 150 °C mit Wasserstoff behandelt.

Gegenstand der vorliegenden Erfindung ist auch die Verwendung der neuen Verbindungen als Korrosionsschutzmittel in Heizölen, Schmierstoffen oder Treibmitteln auf Kohlenwasserstoff-Basis.

Die Hydroformulierung des Cyclododecatriens-1,5,9 ist bereits bekannt (vgl. US-PS 2089904, US-PS 3354229, FR-PS 1411448 oder GB-PS 1161147). Nach diesen Patentschriften können unter Verwendung von Kobaltkatalysatoren aus Cyclododecatrien entsprechende $C_{13}$-Alkohole, Gemische, die Formylcyclodocan neben Formylcyclodocen, Formylcyclodocadien und dem $C_{13}$-Alkohol enthalten, hergestellt werden. Die Ausbeute an Formylcyclododecan übersteigt dabei selbst unter besonders kontrollierten Bedingungen nicht 40% der Theorie.

Als Hauptprodukt werden stets Hydroxymethylcyclododecane gewonnen. Selbst unter Bedingungen, unter denen aus Cyclooctadien-1,5 Formylcyclooctan in 56,7% Ausbeute gewonnen wird, entsteht aus Cyclododecatrien-1,5,9 nahezu ausschliesslich Hydroxymethylcyclododecan.

Die Herstellung von Di- und Tri-Formylcyclododecanen sollte noch grössere Schwierigkeiten erwarten lassen.

Die Verwendung von Kobaltkomplexen mit Trialkylphosphitliganden bei der Hydroformylierung des Cyclododecatrien ergab ebenfalls in erster Linie Hydroxymethylcyclododecan.

Es wurde nun gefunden, dass Aminomethylcyclododecane mit 13 bis 15 C-Atomen, die durch die allgemeine Formel (2) dargestellt werden können,

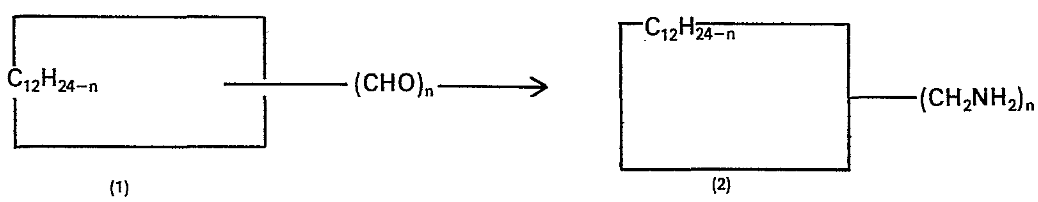

(1) (2)

in der n eine ganze Zahl von 1 bis 3 bedeutet, hergestellt werden können, indem man Cyclododecatrien-1,5,9 in Gegenwart eines Rhodium- und gegebenenfalls Kobalt-haltigen Hydroformylierungskatalysators, der Liganden mit P, N oder S als Hetero-Atom enthält, bei Temperaturen von 80 bis 180 °C mit einem Gemisch aus Kohlenmonoxid und Wasserstoff unter 30 bis 900 bar Druck umsetzt, die entstehenden Formylcyclododecane der allgemeinen Formel (1), in der n eine ganze Zahl von 1 bis 3 ist, aus dem Reaktionsgemisch, insbesondere von dem Hydroformylierungskatalysator abtrennt und in Gegenwart eines Hydrierkatalysators bei 50 bis 150 °C und 10 bis 200 bar Wasserstoffdruck mit einem Ammoniaküberschuss reduktiv aminiert, wobei vor der reduktiven Aminierung auch eine destillative Reindarstellung der im Reaktionsgemisch vorliegenden Formylcyclododecane erfolgen kann. Insbesondere kann bei der gegebenenfalls erwünschten Herstellung von mindestens 2 Aminogruppen aufweisenden erfindungsgemässen Verfahrensprodukten vor der reduktiven Aminierung das monofunktionelle Formylcyclododecan destillativ abgetrennt und gegebenenfalls getrennt einer reduktiven Aminierung zugeführt werden.

Das als Ausgangsprodukt beim erfindungsgemässen Verfahren einzusetzende Cyclododecatrien ist bekannt. Es kann beispielsweise durch Trimerisierung von Butadien mit Metallkatalysatoren, wie in Angewandte Chemie Bd. 69, Seite 397 (1957) beschrieben ist, hergestellt werden. Das Cyclododecatrien-1,5,9 kann in der cis-trans-trans-, und in der trans-trans-trans-Form verwendet werden.

Bei der Hydroformylierung werden Kohlenmonoxid und Wasserstoff in der Regel mindestens im stöchiometrischen Verhältnis, vorteilhaft jedoch im Überschuss, z.B. bis zu 1000 Mol-%, angewandt. Das Gemisch aus Kohlenmonoxid und Wasserstoff enthält Kohlenmonoxid und Wasserstoff in der Regel im Volumenverhältnis von 1:4 bis 4:1, insbesondere im Verhältnis von 2:1 bis 1:2.

Die Hydroformylierung führt man bei einer Temperatur von 80 bis 180 °C durch. Besonders bewährt haben sich Temperaturen von 90 bis 165 °C. Ferner hält man bei der Reaktion einen Druck von 30 bis 900 bar ein. Vorteilhaft wendet man einen Druck im Bereich von 200 bis 400 bar an. Die Reaktionstemperatur wird entsprechend dem gewünschten Reaktionsprodukt eingestellt.

So erhält man im Temperaturbereich von 80 bis 120 °C, bevorzugt den Monoaldehyd, aus dem das Monoamin hergestellt wird, während bei Temperaturen oberhalb 150 °C, bevorzugt das Trisformylcyclododecan gewonnen wird. Die Reaktionszeit ist abhängig von der Reaktionstemperatur. Zur Gewinnung des Trisformylcyclododecans ist es erforderlich, die Konzentration des Cyclododecantriens im Reaktionsgemisch gering zu halten, indem man das Trien nur langsam in das Reaktionsgemisch eindosiert.

Für die Hydroformylierung geeignete Katalysatoren sind Rhodiumkomplexe die einen oder mehrere stickstoff-, phosphor- und/oder schwefelhaltige Liganden besitzen. Bevorzugte, als Katalysatoren verwendete Rhodium komplexe haben die Formeln

$XRh(CO)L_2$,$XRh(CO)L_3$,$RhXL_3$, $[Rh(CO)L_2]_2$, $[Rh(OCOCH_3)(CO)L]_2$, wobei X für ein Chlor-, Brom- oder Jodatom und L für einen organischen Liganden steht. Geeignete organische Liganden können tertiäre organische Phosphine, Phosphite, Dialkylsulfide und tertiäre Amine sein. Geeignete Liganden sind beispielsweise:
Tertiäre organische Phosphine oder organische Phosphite, die als organische Reste gleiche oder verschiedene Alkylreste mit 1 bis 20 Kohlenstoffatomen, Cycloalkylreste mit 5 bis 12 Kohlenstoffatomen, Aralkylreste mit 7 bis 10 Kohlenstoffatomen und mindestens einen Arylrest mit 6 bis 10 Kohlenstoffatomen haben. Die genannten Reste können unter Reaktionsbedingungen inerte Substituenten haben, z.B. 1 bis 2 Hydroxylgruppen, Alkoxy- oder Carboalkoxygruppen mit 1 bis 4 Kohlenstoffatomen, Aminogruppen oder Halogenatome, wie Triphenylphosphin, Diäthylphenylphosphin, Tritolylphosphin, Trinaphthylphosphin, Diphenylmethylphosphin, Diphenylbutylphosphin, Tris-(p-chorphenyl)-phosphin, Tris-(p-carbmethoxyphenyl)-phosphin. Tris-(p-cyanophenyl)-phosphin, Diphenyl-phosphonig-säurephenylester, Benzol-phosphonigsäure-di-phenylester und Triphenyl phosphit,

$P[CH_2CH_2CH_2N(CH_3)_2]_3$,

$P[CH_2CH_2CH_2N(C_2H_5)_2]_3$,

$P(CH_2CH_2CH_2-\underset{H}{N}-iso-C_4H_9)_3$,

$P[CH_2CH_2CH_2N(iso-C_4H_9)_2]_3$,

$(n-C_4H_9)_2PCH_2CH_2N(C_2H_5)_2$,

$P[CH_2N(C_2H_5)_2]_3$,

$P[C_6H_4N(CH_3)_2]_3$,

$P[CH_2CH_2C_6H_4N(C_2H_5)_2]_3$,

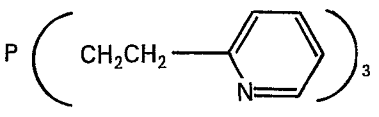

$P[CH_2CH_2CH_2N(ter\cdot C_4H_9)_2]_3$ und

$P[CH_2CH_2CH_2N(iso-C_3H_7)_2]_3$.

Die verwendeten phosphorhaltigen Liganden werden am zweckmässigsten aus der Gruppe der Triarylphosphine, Triarylphosphite und Triarylphosphate ausgewählt, wobei die Triarylphosphine und Triarylphosphite normalerweise am besten geeignet sind. Ebenfalls gemäss der Erfindung verwendbar sind komplexe Liganden in Form von teilweise durch Ferrocen substituierten Triorganophophinen (vgl. die DE-OS 2 617 306). Generell ist jedoch jeder Triorganophosphorligand, der eine bekannte Eignung für rhodiumkatalysierte Hydroformylierungs-Reaktionssysteme besitzt, verwendbar.

Geeignete stickstoffhaltige Liganden sind beispielsweise:
Pyridin, Picoline, Äthylpyridine, N-Methylpyrolidin, N-Methylpyrrol, N,N'-Dimethylpiperazin, Dimethylcyclohexylamin, Triäthylamin, N,N-Dimethylanilin, N-Methylmorpholin, N-Methylindol, Chinolin, Isochinolin, N-Methylpyrrolidon und 3-Dimethylaminopropionitril.

Geeignete schwefelhaltige Liganden sind beispielsweise:
Dibenzylsulfid, Di-n-butylsulfid, Dimethylsulfoxid, Diethylsulfid, Di-(4-Chlor-benzyl)-sulfid, Di-(4-Cyanobenzyl)-sulfid, Bis-(4-Dimethylaminobenzyl)-sulfid, Di-(4-diäthylaminobenzyl)-sulfid, Di-(α-naphthylmethyl)-sulfid, Di-(2,6-dichlorbenzyl)-sulfid, Di-(3,4-dichlorbenzyl)-sulfid, Di-(2-chlorbenzyl)-sulfid, Di-(5,6,7,8-tetrahydronaphthyl-2-methyl)-sulfid, Benzyl-methylsulfid, Benzyl-dodecyl-sulfid, 4-Dimethylaminobenzylmethyl-sulfid, Benzyl-butyl-sulfid, Bis-(4-carboxybenzyl)-sulfid, Di-(4-methylbenzyl)-sulfid, Di-(3-methylbenzyl)-sulfid, Di-(2-methylbenzyl)-sulfid.

Die Methode, nach welcher der katalytische Komplex des Rhodiums mit dem Liganden und Kohlenmonoxid in das Hydroformylierungs-Reaktionssystem eingeführt wird, ist für die Anwendbarkeit unwesentlich. Das Mengenverhältnis des Liganden zum Rhodium im Katalysatorkomplex kann innerhalb eines breiten Bereichs liegen; normalerweise enthält das flüssige Reaktionsmedium jedoch mindestens etwa 1 Mol des Liganden (z.B. Triphenylphosphin) pro Grammatom Rhodium. Der Ligand kann aber auch in hohem Überschuss zugesetzt werden.

Es ist möglich, im Katalysatorsystem das Rhodium teilweise durch Kobalt zu ersetzen.

Der Rhodiumkatalysator wird in einer Menge bei der Umsetzung des Cyclododecatriens mit Kohlenmonoxid und Wasserstoff verwendet, die 1 bis 1000 mg Rh-Metall je kg Cyclododecatrien entspricht. Wird zusätzlich Kobalt als Katalysator verwendet, so kann sich die aufzuwendende Menge Rhodium auf ein Zehntel bis ein Hundertstel verringern. Kobalt wird entsprechend 0,1 bis 10 g Co je kg Cyclododecatrien verwendet.

Insbesondere werden 10 bis 600 mg Rh/kg Cyclododecatrien verwendet. Der Katalysator kann nach den bekannten Methoden wiedergewonnen

und erneut verwendet werden.

Die Hydroformylierung wird in der flüssigen Phase durchgeführt. Es ist möglich, den homogenen Katalysator auf einen festen Träger zu fixieren, wie es beispielsweise von P.I. Davidson e.a. in Catalysis Vol 1, (1976), Seiten 391-393 beschrieben wurde.

Das flüssige Reaktionsmedium kann entweder ein Gemisch von an sich vorhandenen Flüssigkeiten (d.h. Reaktionsprodukten, überschüssigem Ligand etc.) darstellen oder gegebenenfalls auch ein zugesetztes Lösungsmittel sein, das bei den Reaktionsbedingungen inert ist und in dem der homogene Katalysator und der überschüssige Ligand löslich sind. Wenn kein gesondertes Lösungsmittel verwendet wird, enthält das Reaktionsmedium zumeist einen Überschuss des Liganden (z.B. von Triphenylphosphin) sowie Reaktionsprodukte, zu denen insbesondere jene Nebenprodukte gehören, die weniger flüchtig als das Carbonyl-Reaktionsprodukt selbst sind.

Wenn die Umsetzung in Gegenwart einer getrennt zugesetzten Lösungsmittelart durchgeführt wird, kann man dafür in bekannter Weise die verschiedensten inerten Flüssigkeiten verwenden, beispielsweise alkylsubstituierte Benzole, Pyridin bzw. alkylsubstituierte Pyridine, tertiäre Amine, hochsiedende Ester, wie Dialkyldicarboxylate, Triorganophosphate oder Ester von Polyolen (wie Trimethylolpropan oder Pentaerythrit), Ketone, Alkohole, wie die Butanole, Nitrile, wie Acetonitril oder Kohlenwasserstoffe, z.B. gesättigte aliphatische oder cycloaliphatische Kohlenwasserstoffe.

Bevorzugt werden Lösungsmittel verwendet, die auch in der nachfolgenden reduktiven Aminierung verwendet werden können, wie Benzol, Toluol, Xylol, Isopropanol, Methylcyclohexan, Dekalin, Dioxan, Tetrahydrofuran, Äthylenglykolmonoethyläther oder Diäthylenglykoldimethyläther.

Die durch Hydroformylierung gewonnenen Aldehyde können in bekannter Weise, z.B. durch Destillation, vom Katalysator abgetrennt und dann der reduktiven Aminierung unterworfen werden. Oft empfiehlt es sich, beispielsweise bei der Herstellung von reinen Monoaminen oder bei der Herstellung von keine Monoamine aufweisenden Polyaminen, das Hydroformylierungsprodukt vor der reduktiven Aminierung destillativ in die entsprechenden Komponenten zu zerlegen. Es ist ebenso möglich, das Gemisch der Hydroformylierungsprodukte reduktiv zu aminieren und anschliessend Mono- und Polyamine destillativ zu trennen. Bei der reduktiven Aminierung kann das bei der Hydroformylierung gegebenenfalls verwendete Lösungsmittel ebenfalls Verwendung finden. Die reduktive Aminierung wird in Gegenwart eines Hydrierkatalysators und von wenigstens 3 Mol Ammoniak je Mol des Formylcyclododecans durchgeführt. Es ist auch möglich, die reduktive Aminierung ohne Lösungsmittel oder in den erfindungsgemässen Aminomethylcyclododecanen oder in einem grossen Ammoniaküberschuss durchzuführen. Bevorzugt wird ein Molverhältnis Ammoniak/Formylverbindung von mehr als 10. Es kann von Vorteil sein, eine Säure in katalytischer Menge zuzusetzen. Bevorzugt wird der Zusatz von 0,1 bis 3 Gew.-% Phosphorsäure, Propionsäure oder Bernsteinsäure.

Die reduktive Aminierung wird bei Temperaturen von 50 bis 150 °C, insbesondere bei 90 bis 135 °C, durchgeführt. Der Wasserstoffdruck sollte mehr als 10, insbesondere 50 bis 200 bar betragen.

Geeignete Katalysatoren für die reduktive Aminierung sind Hydrierkatalysatoren, die als aktive Komponente Metalle mit den Atomnummern 23 bis 29 in metallischer und/oder oxidischer Form enthalten. Geeignete Katalysatoren sind beispielsweise Nickel- oder Kobalt-Katalysatoren, wie Nickel-auf-Träger, wobei als Träger anorganische Materialien wie Kieselgur, Kieselsäuren, Aluminiumoxide, Silikate, Aluminiumsilikate, Montomorillonit, Zeolithe, Spinelle, Dolomit, Kaolin, Magnesiumsilikate, Zirkonoxid, Eisenoxid, Zinkoxid, Calciumcarbonat, Siliziumcarbid, Aluminiumphosphat, Borphosphat, Asbest oder Aktiv-Kohle und als organische Katalysatorträger natürlich vorkommende oder synthetische Verbindungen mit hohem Molgewicht wie Seide, Polyamide, Polystyrole, Zellstoff oder Polyurethane verwendbar sind, wobei die Träger in Form von Kugeln, Strängen, Fäden, Zylindern, Polygonen oder in Pulverform vorliegen können, Raney-Typ-Katalysatoren, wie Raney-Nickel, W-1-, W-5-, W-6-, W-7-Raney-Nickel wie von H. Adkins, J. Am. Chem. Soc. 69, 3039 (1974) beschrieben, Raney-Kobalt-Katalysatoren, Raney-Kupfer, Raney-Nickel-Eisen, Raney-Kobalt-Nickel, Raney-Kobalt-Eisen, durch Reduktion von Nickel- oder Kobaltsalzen hergestellte Metallkatalysatoren, wie Urushibara-Nickel oder mit Metallalkylverbindungen, Alkalihydriden, Hydrazin, Boranaten oder Borwasserstoff reduzierte Nickel- oder Kobaltsalze, durch Reduktion der Metalloxide oder Metalloxidgemische hergestellte Katalysatoren, die Metalloxide oder -oxidgemische.

Die Reduktion der Metalloxide oder Metallsalze kann auch mit Wasserstoff, gegebenenfalls bei erhöhter Temperatur und erhöhtem Druck oder unter den Bedingungen des Verfahrens oder während des Verfahrens geschehen.

Die Katalysatoren können als Beschleuniger eines oder mehrere der folgenden Elemente in Mengen bis zu 10% enthalten:

Li, Na, Ca, Ba, K, Ag, Be, La, Ce, Ti, V, Nb, Ta, Mo, W, und bis zu 1% der Elemente Ru, Rh, Pd, Au, Ir, Pt.

Besonders bevorzugte Hydrierkatalysatoren sind Raney-Katalysatoren, wie Raney-Nickel, Raney-Kobalt und Raney-Nickel-Eisen.

Für die Herstellung von Di- und Triaminomethylverbindungen kann es vorteilhaft sein, in einem Hydrierautoklaven den Katalysator, Ammoniak und ein Lösungsmittel oder das Endprodukt unter Wasserstoffdruck bei der Hydriertemperatur vorzulegen und das entsprechende Formylcyclododecan gegebenenfalls in einem Lösungsmittel einzupumpen.

Die erfindungsgemässen Amine können aus dem Reaktionsgemisch, nachdem der Hydrierkatalysator in bekannter Weise beispielsweise durch Filtration oder Zentrifugieren entfernt wurde, durch Destillation abgetrennt werden.

Bei den erfindungsgemässen Aminen handelt es sich um Aminomethyl-substituierte Cyclododecane mit 1 bis 3 Aminomethyl-Substituenten pro Molekül. Im allgemeinen fallen die erfindungsgemässen Verfahrensprodukte, falls auf eine Auftrennung der als Zwischenstufe eingesetzten Hydroformylierungsprodukte verzichtet wurde, als Mono-, Di- und Triamine enthaltendes Gemisch an, in welchem der Gehalt an Mono-, Di- bzw. Triaminen beispielsweise - wie bereits ausgeführt - durch Wahl der Hydroformylierungs-Temperatur innerhalb gewisser Grenzen eingestellt werden kann. Die Reindarstellung von Mono-, Di- bzw. Triaminen oder von ausschliesslich Di- und Triamine enthaltenden Gemischen kann durch entsprechende destillative Reindarstellung auf der Zwischenstufe des Hydroformylierungsproduktes erfolgen, da die Zusammensetzung der erfindungsgemässen Verfahrensprodukte der Zusammensetzung dieser Zwischenstufe entspricht. Selbstverständlich ist es jedoch auch möglich, die Zerlegung des Reaktionsgemischs in die einzelnen Komponenten nach Abschluss der reduktiven Aminierung, beispielsweise durch Destillation, durchzuführen. Bei den erfindungsgemässen Di- und Triaminen handelt es sich jeweils um Isomerengemische, deren genaue Zusammensetzung für die technische Verwertbarkeit der erfindungsgemässen Amine ohne Belang ist.

Die erfindungsgemässen Amine stellen wertvolle Korrosionsschutzmittel dar, die wegen ihres hohen Kohlenwasserstoffanteils insbesondere eine ausgezeichnete Verträglichkeit mit Heizölen, Schmierstoffen und Treibmitteln auf Kohlenwasserstoff-Basis aufweisen. Die erfindungsgemässen Di- und Triamine bzw. deren Gemische stellen im übrigen interessante Vernetzer bzw. Kettenverlängerungsmittel für Epoxidharze bzw. NCO-Präpolymere dar, wie sie in an sich bekannter Weise zur Herstellung von Polyurethan-Polyharnstoffen eingesetzt werden.

Beispiel 1

Hydroformylierung des Cyclododecatriens

In einen Autoklaven aus Edelstahl werden der Katalysator, 75 mg Tris-(dibenzylsulfid)-trischlor-Rhodium und 2,4 g Dikobaltoctacarbonyl und 500 g Toluol eingefüllt. Der Autoklav wird mit einem 1:1-Gasgemisch aus Kohlenmonoxid und Wasserstoff mehrmals gespült und dieses Gasgemisch wird bis 100 bar aufgepresst. Man heizt unter Rühren den Autoklaven bis 170 °C auf und ergänzt mit dem gleichen Gasgemisch den Druck bis 200 bar und hält im folgenden den Druck konstant, indem weiter $CO/H_2$ nachgedrückt wird, wenn der Druck abfällt. Nach 1 Stunde wird die Temperatur auf 110 °C gesenkt und im Verlauf von 3 Stunden die Lösung von 500 g Cyclododecatrien-1,5,9 in 1000 g Toluol in den Autoklaven eingepumpt. Nach weiteren 90 Minuten wird das Reaktionsgemisch im Autoklaven abgekühlt, entspannt und mit Stickstoff gespült. Die Reaktionslösung wird filtriert. Bei 1600 Pa wird das Lösungsmittel abdestilliert. Das Reaktionsprodukt wird bei 13 Pa und einer Manteltemperatur von ca. 220 °C über einen Dünnschichtverdampfer destilliert. Die Zusammensetzung des Destillats wird gaschromatographisch untersucht (Säule: 1 m Carbowachs 6000 auf Teflon; Heizrate: 15° $min^{-1}$; 130 bis 260 °C. Die Probe wird mit Tetrahydrofuran verdünnt).

Die Ergebnisse der Versuche der Beispiele 1 bis 6, die wie im Beispiel 1 durchgeführt wurden, wobei jedoch die in der Tabelle 1 aufgeführten Katalysatoren und Lösungsmittel verwendet und die in der Tabelle 1 aufgeführten Reaktionstemperaturen und Reaktionszeiten eingehalten wurden, sind in der Tabelle 1 zusammengefasst.

Aus dem dünnschichtdestillierten Reaktionsgemisch des Beispiels 2 wurde durch Redestillation Bisformylcyclododecan in einer Reinheit von ca. 95% gewonnen, Siedepunkt: 102 °C bei 0,7 Pa. $C_{14}H_{24}O_2$, MG 224,3.

Aus dem Reaktionsgemisch des Beispiels 6 wurde durch Redestillation das Monoformylcyclododecan in über 95% Reinheit gewonnen; Siedepunkt 91 bis 93 °C bei 13 Pa, $n_D^{20}$ 1.4853.

Tabelle 1

| Beispiel Nr. | 1 | 2 | 3 | 4 | 5 | 6 |
|---|---|---|---|---|---|---|
| Katalysator Ligand L | $(\varnothing CH_2)_2S$ 1) | $\varnothing_3P$ | $\varnothing_3P$ | $(\varnothing CH_2)_3N$ | $(\varnothing CH_2)_2S$ | $\varnothing_3P$ |
| g Rh/kg Cyclododecatrien | 0,018 | 0,2 | 0,5 | 0,2 | 0,012 | 0,5 |
| g Co/kg Cyclododecatrien | 1,0 | – | – | – | 0,7 | – |
| Mol-Verhältnis L/Rh | 3 | 6 | 6 | 3 | 3 | 6 |
| Lösungsmittel | Toluol | Toluol | Dioxan | THF 2) | Toluol | Toluol |
| Reaktionsbedingungen | | | | | | |
| Temperatur 3 °C | 110 | 120 | 160 | 160 | 130 | 100 |
| Druck bar | 200 | 300 | 300 | 300 | 200 | 150 |
| Einpumpzeit h | 3 | 3 | 5 | 2 | 3 | 2 |
| Gesamtreaktionszeit h | 4,5 | 4 | 6 | 3 | 4 | 3 |
| Umsatz in % | 100 | 100 | 98,8 | 50,7 | 67,9 | 88,2 |

Tabelle 1 (Fortsetzung)

| Beispiel Nr. | 1 | 2 | 3 | 4 | 5 | 6 |
|---|---|---|---|---|---|---|
| Ausbeute in Mol-% | | | | | | |
| Monoformyl-CD[3] | 13,9 | 9,2 | 0,1 | 43,1 | 46,0 | 59,5 |
| Bisformyl-CD | 57,7 | 71,8 | 13,1 | 4,9 | 21,2 | 19,1 |
| Triformyl-CD | 26,2 | 13,0 | 85,5 | - | 2,0 | - |
| Rückstand in % | 2,8 | 5,0 | | 3,7 | 1,6 | 2,8 |

[1] ∅ = $C_6H_5$–; [2] Tetrahydrofuran; [3] CD = Cyclododecan

Reduktive Aminierung

Beispiel 7

Aminomethylcyclododecan

In einem Rührautoklaven aus Edelstahl werden 235 g Formylcyclododecan, 2 g Essigsäure, 250 g Tetrahydrofuran und 20 g Raney-Kobalt eingefüllt. Der Autoklav wird verschlossen, mit Stickstoff gespült. 300 g Ammoniak werden flüssig in den Autoklaven gepumpt. Unter 80 bar Wasserstoffdruck wird auf 110 °C erhitzt und mit Wasserstoff der Druck bis 120 bar 45 Minuten konstant gehalten. Dann ist die reduktive Aminierung beendet. Aus dem abgekühlten Reaktionsgemisch wird nach Verdampfen des Ammoniaks der Katalysator durch Filtration entfernt und das Lösungsmittel abdestilliert. Man erhält durch Destillation bei 13 Pa eine bei 112 bis 113 °C siedende Fraktion, die zu 98,5% aus Aminomethylcyclododecan besteht, $n_D^{20}$ 1.5012, Val: gef. 200 (theoretisch 197), Ausbeute: 86,7%.

Beispiel 8

In einem Autoklaven werden 50 g Raney-Nikkel, 1000 g Methanol, 2 g Phosphorsäure und, - nachdem der Autoklav verschlossen und mit Stickstoff gespült wurde, - 700 g Ammoniak eingefüllt. Der Autoklav wird auf 95 °C unter Wasserstoffdruck erhitzt, so dass sich ein Druck von 120 bar einstellt. Bei 90 bis 100 °C und 120 bar Druck, der durch Nachpressen von Wasserstoff konstant gehalten wird, wird die Lösung von 500 g eines Gemisches, das bei der Formylierung des Cyclododecatrien-1,3,5 entstanden ist und 7,9 Gew.-% Monoformyl-cyclododecan, 71,6 Gew.-% Bisformyl-cyclododecan und 14,7 Gew.-% Trisformylcyclododecan enthält, in 1000 g Methanol im Verlauf von 90 Minuten eingepumpt. Danach wird noch 10 Minuten bei 105 °C und 120 bar gerührt.

Die übliche Aufarbeitung des Reaktionsgemisches ergaben 435 g Amingemisch, das im Dünnschichtverdampfer bei 25 Pa und 170 °C Wandtemperatur überging. Aufgrund des Gaschromatogramms enthielt dieses Gemisch 8,5% Mono-, 76,3% Di- und 13,4% Tri-(aminomethyl)-cyclododecan.

Aus diesem Gemisch kann durch Redestillation eine bei 1,3 Pa und 122 bis 140 °C siedende Fraktion gewonnen werden, die neben 41% Di- etwa 59% Tri-(aminomethyl)-cyclododecan enthält und frei von Mono-(aminomethyl)-cyclododecan ist.

Das Di-(aminomethyl)-cyclododecan (Reinheit 99%) siedet bei 1,3 Pa und 108 °C; $n_D^{20}$ 1.5163.

**Patentansprüche**

1. Aminomethyl-cyclododecane ausgewählt aus der Gruppe bestehend aus Aminomethyl-cyclododecan, Bis-(aminomethyl)-cyclododecanen, Tris-(aminomethyl)-cyclododecanen und deren Gemischen.

2. Verfahren zur Herstellung von Aminomethylcyclododecanen gemäss Anspruch 1, dadurch gekennzeichnet, dass man Cyclododecatrien-1,5,9 in Gegenwart eines rhodiumhaltigen Katalysators mit Kohlenmonoxid und Wasserstoff bei Temperaturen von 80 bis 180 °C und Drücken von 30 bis 900 bar umsetzt, den Katalysator von dem Hydroformulierungsprodukt abtrennt und die Hydroformulierungsprodukte, gegebenenfalls nach ihrer destillativen Auftrennung in die einzelnen Komponenten, in Gegenwart von Ammoniak und einem Hydrierkatalysator bei 50 bis 150 °C mit Wasserstoff behandelt.

3. Verwendung der Aminomethyl-cyclododecane gemäss Anspruch 1 als Korrosionsschutzmittel in Heizölen, Schmierstoffen oder Treibmitteln auf Kohlenwasserstoff-Basis.

**Claims**

1. Aminomethyl cyclododecanes selected from the group comprising aminomethyl cyclododecane, bis-(aminomethyl)-cyclododecanes, tris-(aminomethyl)-cyclododecanes and mixtures thereof.

2. A process for producing the aminomethyl cyclododecanes claimed in Claim 1, characterised in that cyclododeca-1,5,9-triene is reacted with carbon monoxide and hydrogen in the presence of a rhodium-containing catalyst at temperatures of from 80 to 180 °C and under pressures of from 30 to 900 bars, the catalyst is separated off from the hydroformylation product and the hydroformylation products are treated with hydrogen at from 50 to 150 °C in the presence of ammonia and a hydrogenation catalyst, optionally after separation by distillation into the individual components.

3. The use of the aminomethyl cyclododecanes claimed in Claim 1 as corrosion inhibitors in heating oils, lubricants or motor fuels bases on hydrocarbons.

**Revendications**

1. Aminométhyl-cyclododécanes choisis dans le groupe consistant en aminométhyl-cyclododécane, bis-(aminométhyl)-cyclododécanes, tris-(aminométhyl)-cyclododécanes et leurs mélanges.

2. Procédé de fabrication d'aminométhyl-cyclododécanes selon la revendication 1, caractérisé en ce qu'on fait réagir du cyclododécatriène-1,5,9, en présence d'un catalyseur contenant du rhodium avec de l'oxyde de carbone et de l'hydrogène à des températures de 80 à 180 °C et sous des pressions de 30 à 900 bars, on sépare le catalyseur du produit de l'hydroformylation et on traite les produits de l'hydroformylation, le cas échéant après leur séparation par distillation en les composants individuels, en présence d'ammoniac et d'un catalyseur d'hydrogénation à 50–150 °C, avec de l'hydrogène.

3. Utilisation des aminométhyl-cyclododécanes selon la revendication 1 comme agents de protection contre la corrosion dans des huiles de chauffage, des lubrifiants ou des agents de propulsion à base d'hydrocarbures.